# EUROPEAN PATENT SPECIFICATION

(11) **EP 1 289 571 B1**
(45) Date of publication and mention of the grant of the patent: **21.07.2004**
(21) Application number: 01955279.3
(22) Date of filing: 11.05.2001
(51) Int. Cl.: A61K 51/04, A61K 51/08

(54) **PROCHELATORS OF RADIOMETAL LABELED MOLECULES**
PROCHELATORS VON RADIOMETALL MARKIERTEN MOLEKÜLEN
COMPOSES DE PROCHELATION POUR LA PREPARATION DE MOLECULES MARQUEES PAR UN RADIOMETAL PRESENTANT DE MEILLEURES PROPRIETES BIOLOGIQUES

(30) Priority: 12.05.2000 EP 00110084
(43) Date of publication of application: 12.03.2003
(73) Proprietor: University Hospital, 4031 Basel (CH)
(72) Inventor: MAECKE, Helmut, R., D-79541 Lorrach (DE); EISENWIENER, Klaus, D-79576 Weil (DE); POWELL, Pia, CH-4052 Basel (CH)
(86) International application number: PCT/EP2001/005483
(87) International publication number: WO 2002/024235

(56) References cited:
- HEPPELER, A. ET AL: "Radiometal-labelled macrocyclic chelator-derivatized somatostatin analogu with superb tumour-targeting properties and potential for receptor-mediated internal radiotherapy" CHEM.--EUR. J. (1999), 5(7), 1974-1981 , XP001073647
- EISENWIENER, KLAUS-PETER (1) ET AL: "Synthesis and peptide coupling of a new DOTA-based prochelator, forming neutral complexes with yttrium-90 and indium-111." JOURNAL OF LABELLED COMPOUNDS AND RADIOPHARMACEUTICALS, (MAY, 2001) VOL. 44, NO. SUPPLEMENT 1, PP. S694-S696. PRINT. MEETING INFO.: FOURTEENTH INTERNATIONAL SYMPOSIUM ON RADIOPHARMACEUTICAL CHEMISTRY INTERLAKEN, SWITZERLAND JUNE 10-15, 2001 , XP001073649
- EISENWIENER, K.-P. ET AL: "A convenient synthesis of novel bifunctional prochelators for coupling to bioactive peptides for radiometal labelling" BIOORGANIC & MEDICINAL CHEMISTRY LETTERS (2000), 10(18), 2133-2135 , XP002197765

## Description

The present invention relates to a convenient synthesis of novel bifunctional prochelators for coupling to bioactive peptides for radiometal labeling and to the radiometal labeled peptides that can be prepared while using these novel prochelators.

DOTA (1,4,7,10-tetrakis(carboxymethyl)-1,4,7,10 tetraazacyclo dodecane) and its derivatives constitute an important class of chelators for biomedical applications as they accommodate very stably a variety of di- and trivalent metal ions. Gd(DOTA)⁻¹ is an important MRI (Magnetic Resonance Imaging) contrast agent and as bifunctional versions DOTA is used in radioimmunotherapy.

An emerging area is the use of chelator conjugated bioactive peptides for labeling with radiometals in different fields of diagnostic and therapeutic nuclear oncology. For their convenient and high yield synthesis, prochelators (compounds which become chelators upon deprotection) are necessary which are compatible with the solid and solution phase peptide synthetic procedures. Up till now such prochelators have not been available.

It is therefore the object of the present invention to provide a novel class of prochelators which are compatible with solid and solution phase peptide synthesic procedures, which prochelators can be used for the coupling of chelators to bioactive effector molecules, such as peptides.

According to the invention bifunctional macrocyclic synthons (prochelators) are provided based on DOTA, TRITA, TETA or structures comprising not 4, but 5 or 6 N atoms, which synthons are differentially protected and compatible with solid phase peptide synthesis procedures for labeling with hard Lewis acid radiometals.

Accordingly, the invention relates to polyazamacrocyclic compounds for radiometal labeling, comprising an Nₙ system, wherein n is 4, 5 or 6, with varying ring size, and wherein at least one of the N atoms is substituted with a free carboxylate group for coupling to an amino function in a bioactive effector molecule, while all N atoms carry a protected sidechain. After coupling of the bioactive effector molecule the protected sidechains can be deprotected to expose the chelating functions for labeling.

Particular compounds of the invention have the general formula:

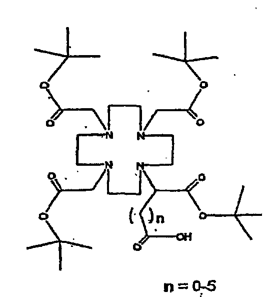

An example of such a compound is 1-(1-carboxy-3-carbotertbutoxypropyl)-4,7,10(carbotertbutoxymethyl)-1,4,7,10-tetraazacyclododecane (DOTAGA(tBu)4).

Compounds of the invention are prepared as follows. Starting from a relevant amino acid, the α-bromo-derivative thereof is synthesized. This derivative is orthogonally protected (tBu, Bzl). This alkylating agent will be reacted with cyclen, cyclam etc. to form a 1:1 adduct followed by tris-alkylation with bromoacetic acid tert-butylester and catalytic hydrogenation with H₂/Pd.

The synthon is monoreactive, carrying a free carboxylate group for coupling to the N-terminal end of the peptide and can be coupled to any biomolecule which then after deprotection can be labeled with a multitude of radiometals known to the person skilled in the art.

The invention relates more in particular to chelating compounds for radioactive labeling of bioactive molecules, which chelating compounds have the general formula: 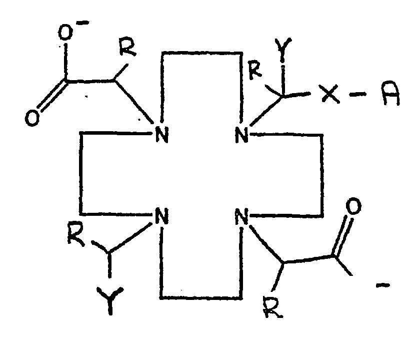 in which:
both Y groups may be positioned either trans as shown or cis;
A is an effector molecule, such as a peptide, in particular octreotide, CCK, substance P, gastrine, a protein, in particular an antibody or enzyme, sugars or radiosensitizing agents, like doxorubicin;
R is a hydrogen, a C₁-C₃ alkyl or an alcohol;
X is a spacer, in particular (CH₂)ₙ-X', in which n is 1-10 and X' is COOH, NH₂, SH, OH or O-halogen, in which halogen is in particular Br, I or Cl
or X is a molecule of the formula 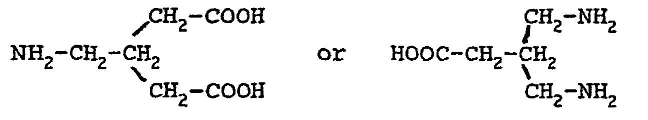
Y is COO⁻, CH₂CONH₂, CH₂CH₂OH.

In principle, the invention relates to every possible combination of the above identified substituents whether or not they are explicitly described herein in a specific combination or not.

Instead of an N₄ system the compound may comprise a N₅ or N₆ system and can be used for different natural and unnatural amino acids, such as protected cystein etc. The list of natural and unnatural amino acids is known to the person skilled in the art.

One of the new DOTA-based bifunctional prochelators, is 1-(1-carboxy-3-carbotertbutoxypropyl)-4,7,10(carbotertbutoxymethyl)-1,4,7,10-tetraazacyclododecane (DOTAGA(tBu)4 (6d)), which serves herein as a model compound. It should be noted that other compounds of the invention can be prepared in an analogous manner. The invention is not limited to compound **6d** alone.

Other embodiments of the invention are listed in Table 3.

The 5 step synthesis of **6d** is described in the Examples. It has an overall yield of about 20%. The coupling of 6d to a bioactive peptide on solid phase is exemplified with use of a CCK-B (cholecystokinin) analogue.

Described herein are the synthetic steps towards bifunctional orthogonally protected prochelators for coupling to the N-terminus of bioactive peptides or other useful amino functions in biomedical applications. The DOTA-derived chelator should provide 4 intact carboxylic acid functions besides the macrocyclic tetraazacyclododecane ring for a stable and efficient binding of metal ions and a function for biomolecule coupling.

The strategy includes the synthesis of an orthogonally protected bromo-alkyl-dicarboxylic acid diester for the monoalkylation of cyclen(1,4,7,10-tetraazacyclododecane). The synthesis of compound **6** (n=1, 2) is a 5 step procedure starting from the commercially available aspartic (**1b**) or glutamic acid-4-(5) benzyl ester (**1d**) (Scheme 1) using a method analogous to Holmberg (Chemische Berichte 1927, 60, 2197-2205) followed by tert-butylation using tert-butyltrichloroacetimidate (TBTA) as reagent (Armstrong, A. et al., Tetrahedron Lett. 1988, 29, 2483-2486).

The monoalkylation of cyclen, the crucial step, showed strongly differing yields depending on the bromo-alkyl-dicarboxylic acid diester (**3a-d**) used (Table 1).

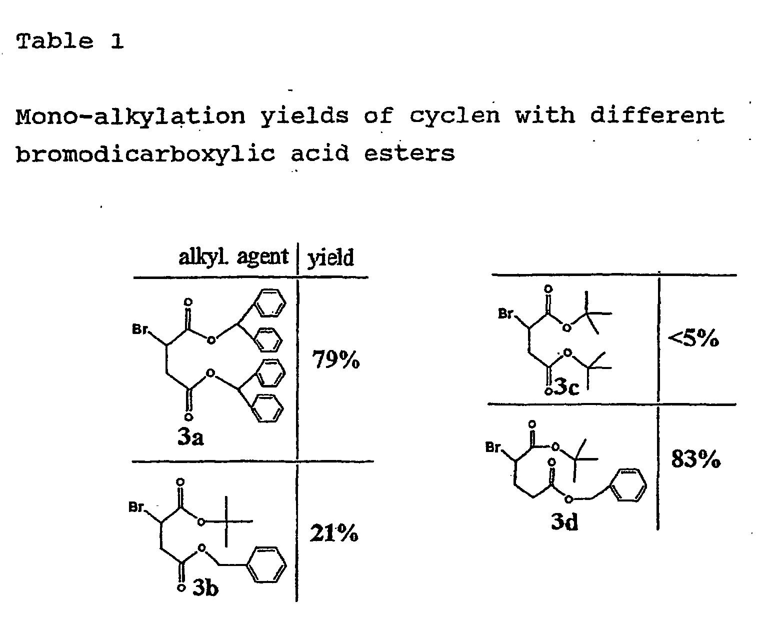

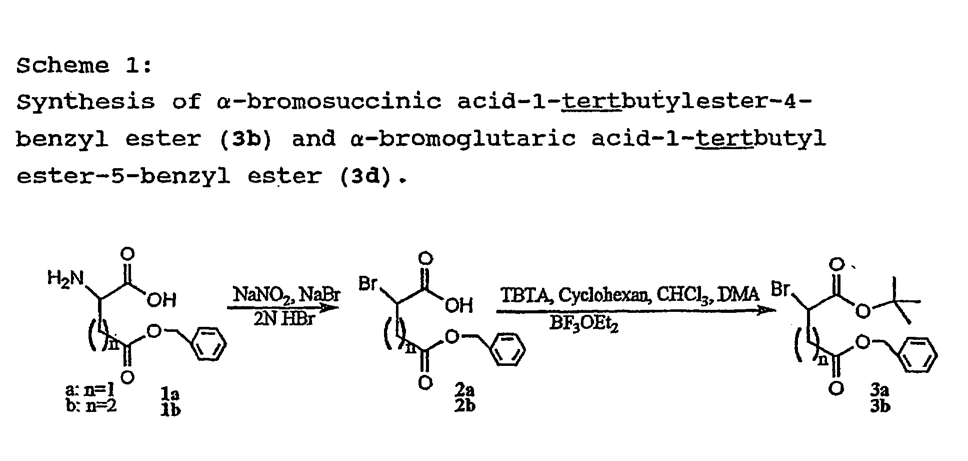

In earlier studies (André et al., Chem. Eur. J. 1999, 5, 2977-2982) the strategy was to use metals as protecting groups. In that work it was attempted to introduce succinic acid-di-tert-butylester (3c) and yields below 5% were found for the monoalkylation with the elimination product fumaric acid-di-tert-butylester as the main product. Interestingly the corresponding diphenylmethyl diester (3a) gave high monoalkylation yields and negligible elimination. With the homologous 2-bromoglutaric-1-tertbutyl-5-benzylester (**3d**), no elimination product was found, obviously because no conjugated pi-system could be formed.

The remaining nitrogens were alkylated by use of three equivalents of bromoacetic acid-tertbutyl ester in CHCl₃/K₂CO₃. 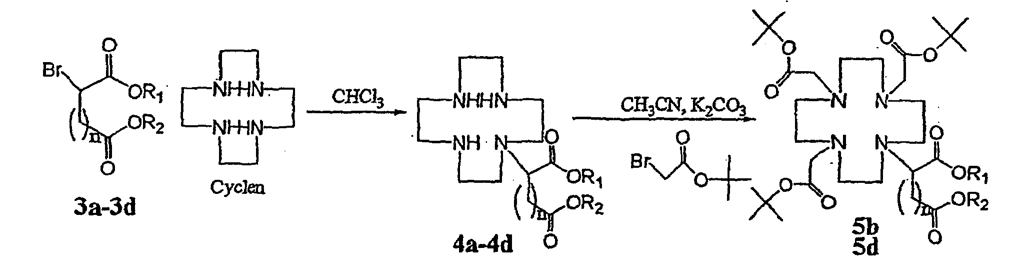 Deprotection of the benzyl ester group was performed with H2/Pd/C. 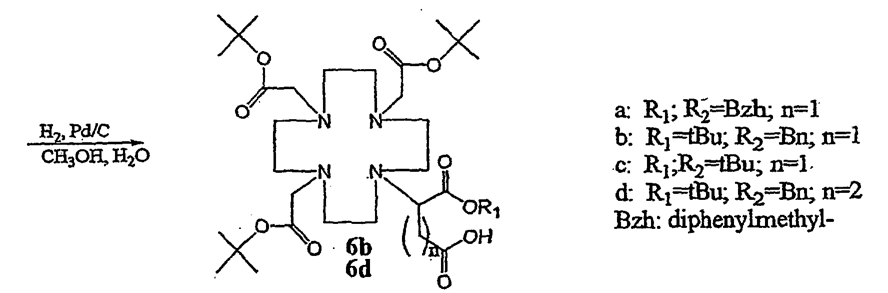 The overall yield of 1-(1-carboxy-3-carbotertbutoxypropyl)-4,7,10(carbotertbutoxymethyl)-1,4,7,10-tetraazacyclododecane (DOTAGA(tBu)₄) (**6d**) over 5 steps was about 20% and of 1-(1-carboxy-2-carbotertbutoxyethyl)-4,7,10(carbotertbutoxymethyl)-1,4,7,10-tetraazacyclododecane (DOTASA(tBu)₄) (**6b**) only about 2%.

The convenient use of **6d** is exemplified by its coupling to the CCK-B analogue D-Asp-Tyr-Nle-Gly-Trp-Nle-Asp-Phe-NH2 (7) attached to Rink-amide resin using HATU (O-(7-azabenzotriazol-1-yl)-N,N,N',N'-tetramethyluroniumhexafluorophosphate) as coupling reagent. After deprotection, (18h, rt, TFA:phenol:thioanisol:water 85:5:5:5) DOTAGA-7 was obtained in high yield and showed superior properties in comparison to other radiolabeled CCK-B analogues.

According to the invention it was thus found that the new type of prochelators in general and prochelator **6d** in particular, have widespread utility in the field of metalloradiopeptides, other radiolabeled biomolecules and for the synthesis of Gd³⁺ based MRI contrast agents. DOTAGA will allow to label with different radiometals for both diagnostic (¹¹¹In, ^{67/68}Ga) and internal radiotherapeutic applications (⁹⁰Y, ¹⁷⁷Lu).

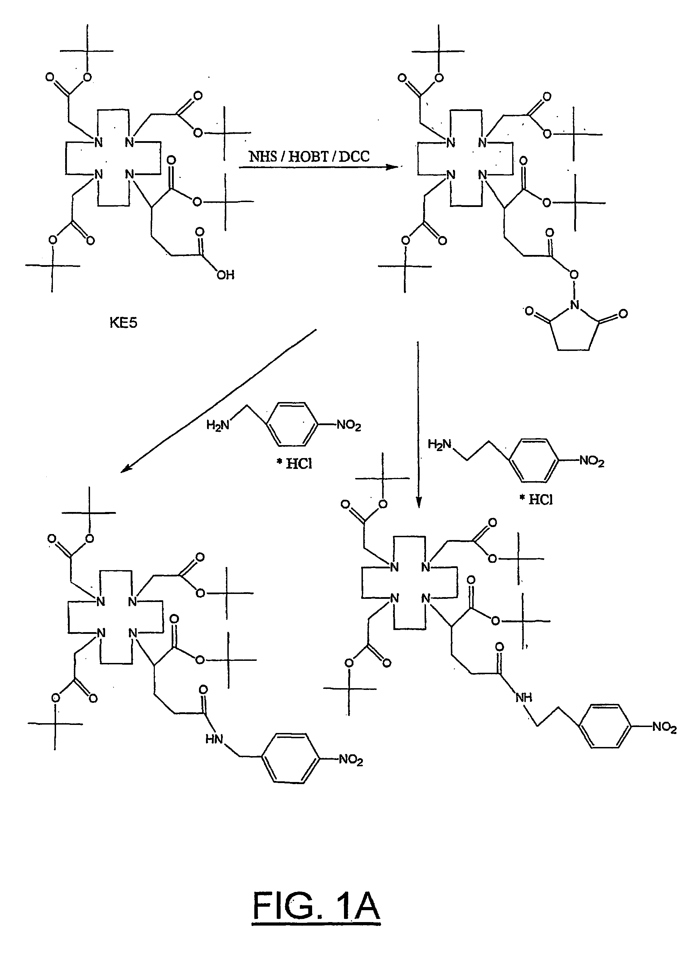

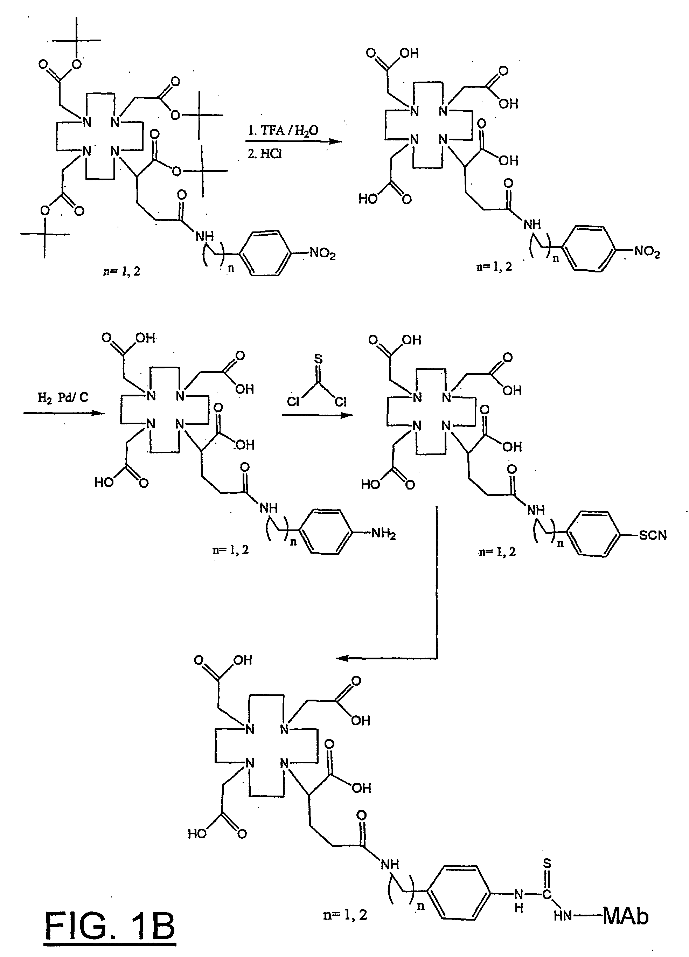

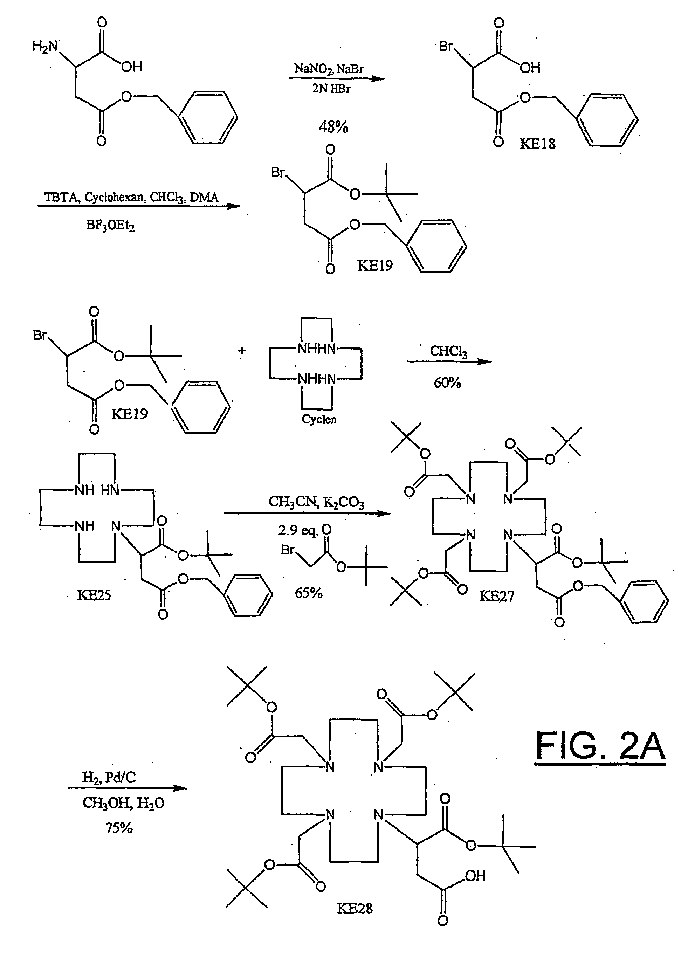

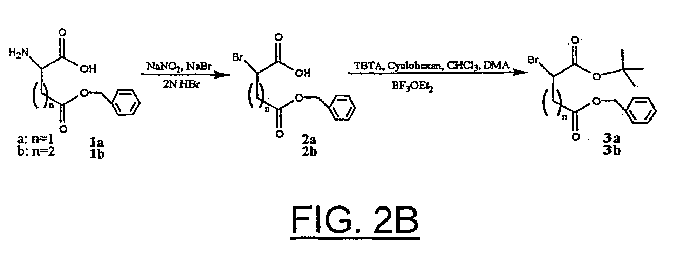

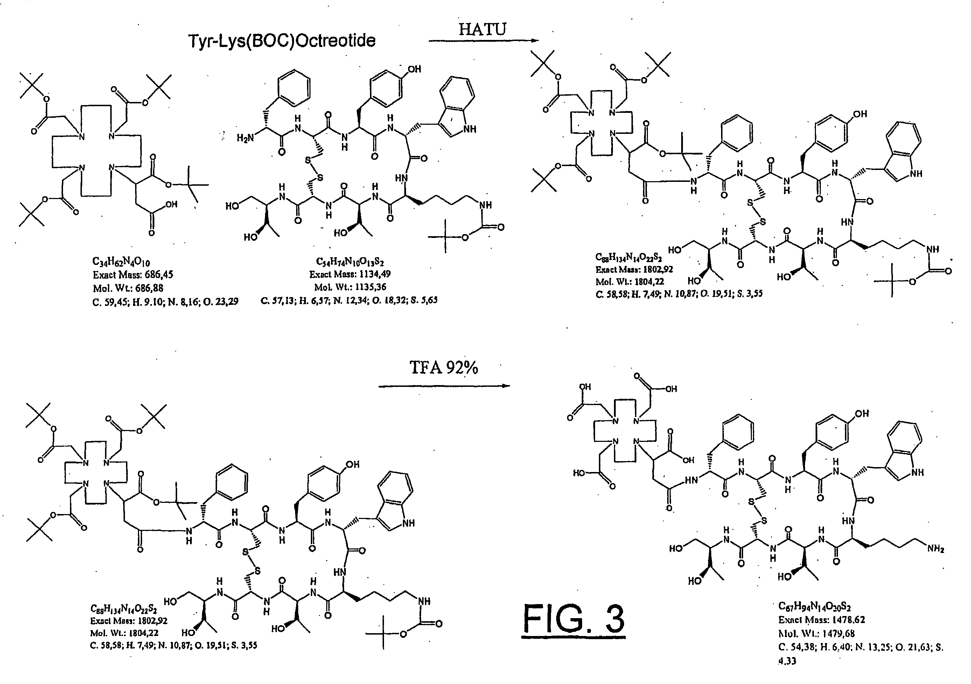

The present invention will be further illustrated in the examples that follow. Synthetic routes for the preparation of the compounds of the invention are given in Figures 1-3.

### EXAMPLES

### EXAMPLE 1

### Typical procedure for the reaction of 1 to 2

To a solution of 6 g (25.9 mmol) L-glutamic acid-5benzylester (**1d**) and 9.1 g (88.5 mmol) sodium bromide in 45 ml aqueous IN hydrobromic acid (46 mmol) cooled to 0°C was added portionwise 3.175 g (46 mmol) sodium nitrite. After stirring for 2h at 0°C 2.25 ml concentrated sulfuric acid was added followed by diethylether.

The water phase was extracted 3 times with diethylether. The combined organic phases were extracted 4 times with brine, dried over Na₂SO₄ and concentrated.

The crude product was purified by chromatography (silica gel 60; hexane/EtOAc 3:1 to 2:1) and obtained as a yellow oil in a yield of 4.8 g (63%). ¹H-NMR (300 MHz, CDCl₃, SiMe₄): 10.1 (1H, COOH; 7.3 (m, 5H, Ar); 5.15 (s, 2 H, CH₂-Ph); 4.4 (dd, ³J= 5.7, 1H, CHBr); 2.6 (t, ³J = 6.8, 2H, CH₂-COOBzl); 2.5-2.2 (m, 2H, CHBr-CH₂-CH₂); ¹³C-NMR (75 MHZ, CDCl₃, SiMe₄): 174.5 (COOH); 171.9 (COOBzl); 135.5 (CH₂C(Ar)); 128.6, 128.4, 128.3 C(Ar)); 66.8 (O-CH₂-Ar); 44.1 (HCBr); 31.4 (HCBr-CH₂); 29.4 (CH₂COOBzl; EI-MS m/z (intensity): 302, 300 (12, [M]⁺); 91 (100, [Bzl]⁺).

### EXAMPLE 2

### Typical procedure for the reaction of 2 to 3

To a solution of 4.8 g (15.9 mmol) **2d** in 20 ml CHCl₃ a solution of 6.26 ml (34.1 mmol) TBTA (tert-butyltrichloroacetimidate) in 20 ml cyclohexane was added dropwise over 20 min. During the addition a white precipitate formed, which was dissolved by the addition of 3.5 ml of DMA followed by 320 µl boron trifluoride ethyl etherate as catalyst. The reaction mixture was stirred for 3 days at Room Temperature (RT).

The mixture was concentrated and the remaining DMA phase was extracted 3 times with 30 ml hexane. The hexane phase was evaporated and the residue chromatographed over silica gel 60 (Hexane/EtOAc 20:1 later 9:1) affording 3.5 g (61%) of a colourless liquid. ¹H-NMR (300 MHz, CDCl₃, SiMe₄): 7.4 (m, 5H, Ar); 5.15 (s, 2H, CH₂-Ph); 4.35 (dd, 1H, CHBr); 2.6 (td, 2H, CH₂-COOBzl); 2.5-2.2 (m, 2H, CHBr-CH₂-CH₂); 1.5 (s, 9H, C(CH₃)₃). ¹³C-NMR (75 MHZ, CDCl₃, SiMe₄): 172.4 COOBzl); 168.7 (COOtBu); 136.1 (CH2C(Ar)); 129.0, 128.8, 128.7 (C(Ar)); 83.1 (C(CH₃)₃); 67.0 (OCH₂-Ar); 47.1 (HCBr); 32.0 (HCBr-CH₂); 30.1 (CH₂COOBzl); 28.1 (CCH₃)₃); EI-MS m/z (intensity): 302, 300 (18, [M-C₄H₉]⁺); 57 (100, [C₄H₉]⁺).

### EXAMPLE 3

### General procedure of the monoalkylation of cyclen

A solution of 870 mg (2.44 mmol) a-bromoglutaric acid-1-tert-butylester-5-benzylester (3d) in CHCl₃ was added dropwise over a period of 1h to a solution of 885 mg (4.9 mmol) cyclen in 4 ml CHCl₃. The mixture was stirred for 2 days at room temperature and concentrated to a brown oil. The crude product was purified by column chromatography (silica gel 60; ethanol/NH₃ 95:5), yield 920 mg (83%) of a colourless oil.

¹H-NMR (300 MHz, CDCl₃, SiMe₄): 7.35 (m, 5H, Ar); 5.1 (s, 2H, CH₂-Ph); 3.25 (dd, 1H, CHBr); 2.9-2.5 (m, 18H, NCH₂, CH₂COOBzl); 2.2-1.85 (m, 2H, CHN-CH₂-CH₂); 1.45 (s, 9H, C(CH₃)₃); ¹³C-NMR (75 MHZ, CDCl₃, SiMe₄): 173.1 (COOBzl); 171.5 (COOtBu); 135.8 (CH₂C(Ar)); 128.5, 128.3, 128.2 (C(Ar)); 81.4 (C(CH₃)₃); 66.2 (O-CH₂-Ar); 63.5 (HCNCH₂); 48.8, 48.0, 46.5, 45.6 (NCH₂CH₂N); 30.6 (CH₂COOBzl); 28.2 (C(CH₃)₃); 24.5 (HCN-CH₂); EI-MS m/z: (intensity): 449.3 (56, [M+H]⁺; 245.8 (100, [M+ CH₃CN+2H]⁺⁺).

### EXAMPLE 4

### Synthesis of 1-(1-carbobenzyloxy-3-carbotertbutoxypropyl)-4,7,10-(carbotertbutoxymethyl)-1,4,7,10-tetraazacyclododecane (5d)

A suspension of 1.1 g (5.6 mmol) bromoacetic acid-tert-butylester, 1.02 g (2.27 mmol) 1-(1-carbobenzyloxy-3-carbotertbutoxypropyl)1,4,7,10-tetraazacyclododecane (**4d**), and 2.63 g (19.1 mmol) of dry potassium carbonate in 10 ml dry acetonitrile was stirred for 18 h at RT and was filtrated afterwards over Celite and evaporated to dryness.

The crude product was purified by column chromatography (silica gel 60; CH₂Cl₂/EtOH 9:1 followed by EtOH/NH₃ 95:5) yield 1.3 9 (73%) of a yellow oil (**5d**). ¹H-NMR (300 MHz, CDCl₃, SiMe₄): 7.35 (m, 5H, Ar); 5.1 (s, 2H, CH₂-Ph); 3.6-1.9 (m, 27H, CHN, NCH₂, CH₂COOBzl, CHNCH₂-CH₂, CH₂COOC(CH₃)₃); 1.45 (s, 36H, C(CH₃)₃); ¹³C-NMR (75 MHZ, CDCl₃, SiMe₄): 174.6 (COOBzl); 172.9, 172.8, 172.6 (COOtBu); 135.6 (CH₂C(Ar)); 128.5, 128.3, 128.2 (C(Ar)); 82.4, 81.8, 81.8 (C(CH₃)₃); 66.3 (O-CH₂-Ar); 55.8, 55.7, 55.4, 52.6, 52.3, 50.3, 48.5, 48.1, 47.1, 44.3 (13C, HCNCH₂, NCH₂CH2_{N}, CH₂COOtBu, CH₂COOBzl); (NCHCH₂CH₂); 28.0, 28.0, 27.8, 27.6 (C(CH₃)₃); EI-MS m/z (intensity): 813.6 (22, [M+Na]⁺); 791.6 (38, [M+H]⁺); 396.5 (100, [M+2H]⁺⁺).

### EXAMPLE 5

### Synthesis of DOTAGA(tBu)4 (6d)

600 mg (0.76 mmol) **5d** was dissolved in methanol, and 30 mg Pd/C suspended in 1 ml H₂O was added. The mixture was hydrogenated for 2 days, filtrated over Celite and evaporated to dryness. The crude product was chromatographed on silica gel 60 (EtOH/NH₃ 95:5) to obtain 470 mg (84.6%) of a white solid (**6d**). ¹H-NMR (300 MHz, CDCl₃, SiMe₄): 6.5 (br, 1H, COOH); 3.6-2.0 (m, 27H, CHN, NCH₂, CH₂COOH, CHN-CH₂-CH₂, CH₂COOC(CH₃)₃); 1.45 (s, 36H, C(CH₃)₃); ¹³C-NMR (75 MHZ, CDCl₃, SiMe₄): 175.2 (COOH); 175.0, 172.9, 172.8, 172.6 (COOtBu); 82.4, 82.1, 81.9 (C(CH₃)₃); 55.8, 60.1 (NCHCOOtBu); 55.9, 55.8, 55.6, 52.7, 52.6, 52.5, 48.6, 48.5, 48.2, 47.1, 44.3 (12C, NCH₂CH₂N, CH₂COOtBu, CH₂COOH); 33.4 (NCHCH₂CH₂); 27.9, 27.8 (C(CH₃)₃); EI-MS m/z (intensity): 723.5 (27, [M+Na]⁺); 701.5 (68, [M+H]⁺); 351.4 (100, [M+2H]⁺⁺).

### EXAMPLE 6

### Preparation of DOTAGA-7

Compound **6d** was coupled to the CCK-B analogue D-Asp-Tyr-Nle-Gly-Trp-Nle-Asp-Phe-NH2 (**7**) attached to Rink-amide resin using HATU (O-(7-azabenzotriazol-1-yl)-N,N,N',N'-tetramethyluroniumhexafluorophosphate) as coupling reagent.

After deprotection, (18h, rt, TFA:phenol:thioanisol:water 85:5:5:5) DOTAGA-7 was obtained in high yield and showed superior properties in comparison to other radiolabeled CCK-B analogues.

The data of the DOTAGA-CCK-B analogue (DOTAGA-7) were as follows: Yield: 12.7 mg, HPLC purity >95%, (+) EI-MS m/z (intensity): 1486.1 (48, [M+H]⁺); 743.7 (60, [M+2H]⁺⁺); (-) EI-MS m/z (intensity): 1484.0 (28, [M+H]⁻); 741.8 (90, [M+2H]⁻⁻).

### EXAMPLE 7

### Biological properties

It has been found that when using the prochelators of the invention for preparing biologically active molecules, these molecules have better biological properties than molecules prepared with other (pro)chelators. The advantages for example are a better labeling yield according to the following table 2:

**Table 2**

| | amount of radioactive label | labeling efficiency of DOTATOC peptide | labeling efficiency of DOTA3-peptide (DOTAGA) (invention) |
|---|---|---|---|
| 5 µg of the compound to be labeled | 5 mCi ⁹⁰Y | 99.5% | 99.9% |
| | 10 mCi ⁹⁰Y | 95% | 100% |
| | 20 mCi ⁹⁰Y | 92% | 99.9% |

In addition, the stability is higher. The reaction of ⁹⁰Y-DOTATOC or ⁹⁰Y-DOTAGA at 37°C with the chelator DTPA results in ⁹⁰Y-DTPA. The half life of this reaction is 23 hours for DOTATOC and 79 hours for DOTAGA.

Table 3 shows various biological properties of the compound of the invention Y-DOTA3TOC and Y-DOTAta.13TOC. The radiometal is not shown.

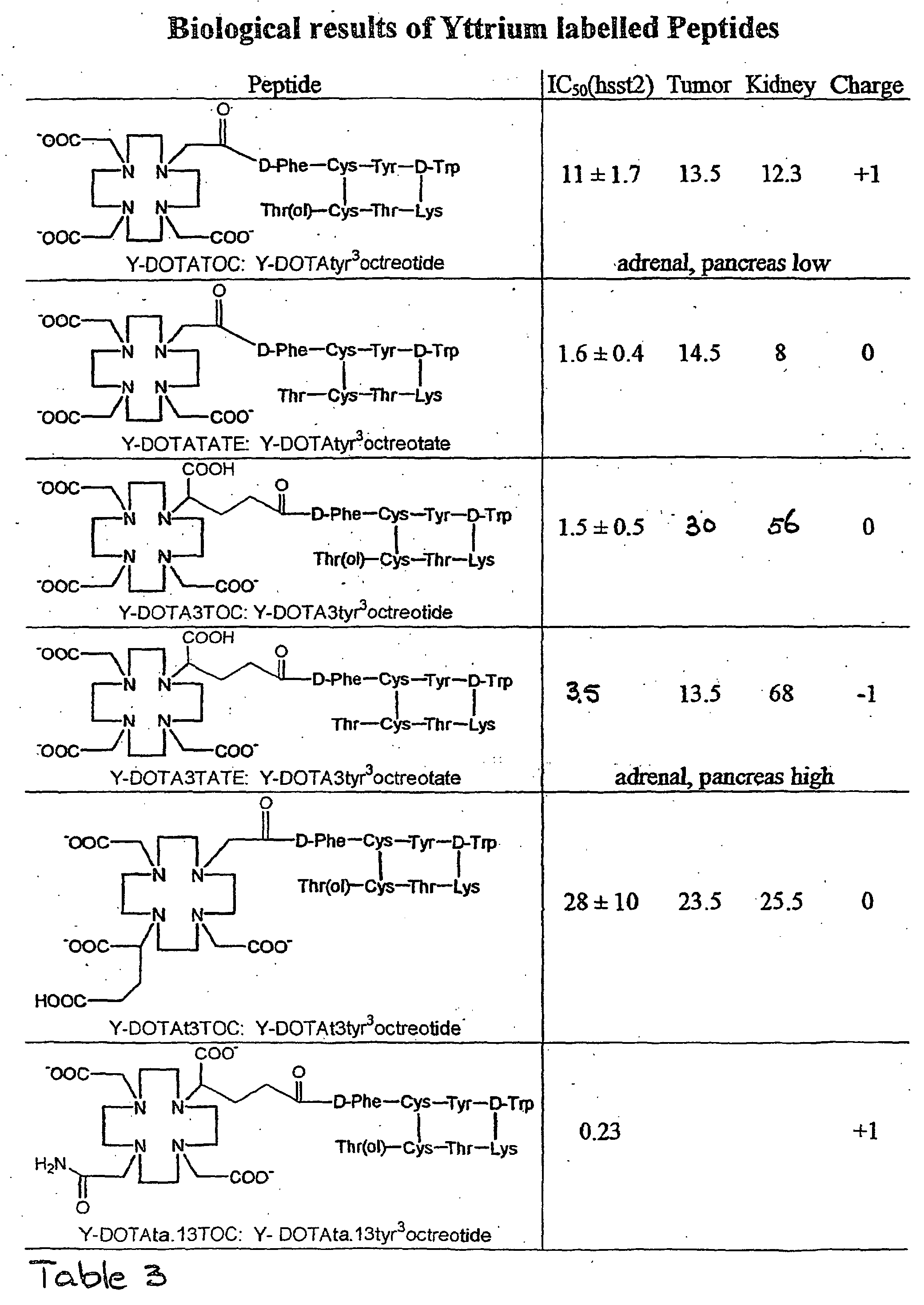

## Claims

1. Polyazamacrocyclic compounds for radiometal labeling, comprising an Nₙ system, wherein n is 4, 5 or 6, with varying ring size, and wherein at least one of the N atoms is substituted with a free carboxylate group for coupling to an amino function in a bioactive effector molecule, while all N atoms carry a protected sidechain.

2. Compound as claimed in claim 1 having the general formula:

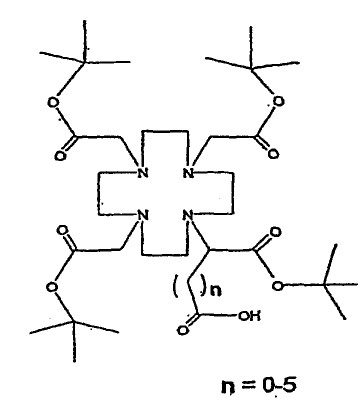

3. Compound as claimed in claim 1 or 2, which compound is 1-(1-carboxy-3-carbotertbutoxypropyl)-4,7,10(carbotertbutoxymethyl)-1,4,7,10-tetraazacyclododecane (DOTAGA(tBu)4).

4. Chelating compounds for labeling bioactive molecules with a radiometal, having the general formula: 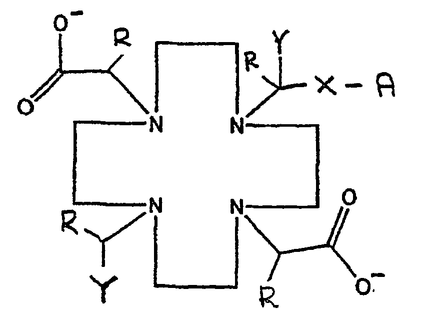 in which:
both Y groups may be positioned either trans as shown or cis;
A is an effector molecule, such as a peptide, in particular octreotide, CCK, substance P, gastrine, a protein, in particular an antibody or enzyme, sugars or radiosensitizing agents, like doxorubicin;
R is a hydrogen, a C₁-C₃ alkyl or a alcohol;
X is a spacer, in particular (CH₂)ₙ-X', in which n is 1-10 and X' is COOH, NH₂, SH, OH or O-halogen, in which halogen is in particular Br, I or Cl
or a molecule of the formula 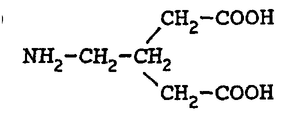 or of the formula 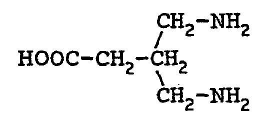 Y is COO⁻, CH₂CONH₂, CH₂CH₂OH,
optionally complexed with a radiometal.

5. Compounds as claimed in claim 4, wherein R is hydrogen, n is 1, X' is COOH, Y is COO⁻ and A is as defined in claim 3.

6. Compound as claimed in claim 5, wherein R is hydrogen, n is 1, X' is COOH, Y is COO⁻ and A is octreotide or octreotate.

7. Compound as claimed in claim 4, wherein R is COOH, n is 1, X' is COOH, Y is COO⁻ and A is as defined in claim 3.

8. Compound as claimed in claim 7, wherein R is COOH, n is 1, X' is COOH, Y is COO⁻ and A is octreotide or octreotate.

9. Compounds as claimed in claim 4, selected from the group consisting of DOTAtyr³octreotide, DOTAtyr³octreotate, DOTA3tyr³octreotide, DOTA3tyr³octreotate, DOTAt3tyr³octreotide, DOTAta.13tyr³octreotate.

10. Use of compounds as claimed in claims 1-3 for the preparation of compounds as claimed in claims 4-9.

11. Method for the preparation of radiometal labeled bioactive molecules, comprising the steps of:
a) synthesizing compounds as claimed in claims 1-3 having protected side chains on the N atoms and a free carboxylate group;
b) coupling a bioactive molecule to the free carboxylate group;
c) deprotecting the protected side chains; and
d) labeling the chelator structure thus obtained with a desired radiometal.

12. Compounds as claimed in claims 4-9 labeled with a radiometal for use in diagnosis and therapy.

13. Use of compounds as claimed in claims 4-9 labeled with a radiometal for the preparation of a diagnostic or therapeutical composition for treatment of various diseases.

14. Use as claimed in claim 13, wherein the radiometal label is ⁹⁰Y.

## Patentansprüche

1. Makrocyclische Polyazaverbindungen zur Markierung mit radioaktiven Metallen, enthaltend ein Nₙ-System, wobei n für 4, 5 oder 6 steht, mit verschiedenen Ringgrößen, und wobei wenigstens eines der N-Atome für die Kupplung an eine Aminofunktion in einem biologisch aktiven Effektormolekül durch eine freie Carboxylatgruppe substituiert ist, während alle N-Atome eine geschützte Seitenkette tragen.

2. Verbindungen nach Anspruch 1 mit der allgemeinen Formel:

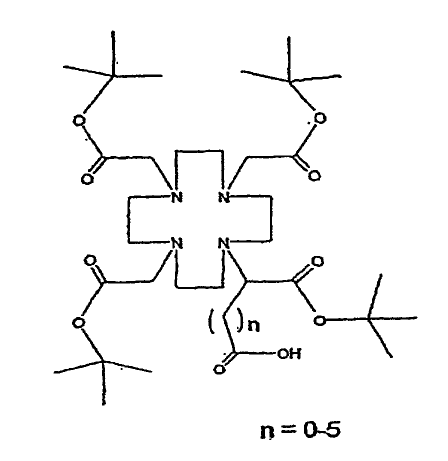

3. Verbindung nach Anspruch 1 oder 2, bei der es sich um 1-(1-Carboxy-3-carbo-tert.-butoxypropyl)-4,7,10-tri(carbo-tert.-butoxymethyl)-1,4,7,10-tetraazacyclododecan (DOTAGA (tBu)4) handelt.

4. Chelatbildner zur Markierung von biologisch aktiven Molekülen mit einem radioaktiven Metall, mit der allgemeinen Formel: 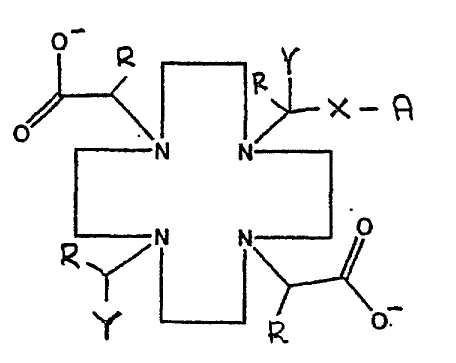 wobei:
die beiden Y-Gruppen entweder, wie gezeigt, trans oder cis angeordnet sein können;
A für ein Effektormolekül wie ein Pep tid, insbesondere Octreotid, CCK, Substanz P oder Gastrin, ein Protein, insbesondere einen Antikörper oder ein Enzym, einen Zucker oder ein radiosensibilisierendes Mittel wie Doxorubicin steht;
R für Wasserstoff, C ₁-C₃-Alkyl oder einen Alkohol steht;
X für einen Spacer, insbesondere (CH ₂)ₙ-X', steht, wobei n für 1-10 steht und X' für COOH, NH₂, SH, OH oder O-Halogen steht, wobei es sich bei dem Halogen insbesondere um Br, I oder Cl handelt,
oder ein Molekül der Formel 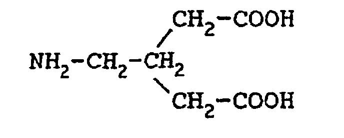 oder der Formel 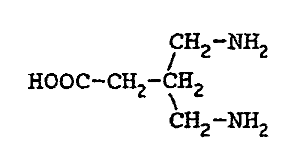 steht,
Y für COO⁻, CH₂CONH₂ oder CH₂CH₂OH steht,
gegebenenfalls komplexiert mit einem rad ioaktiven Metall.

5. Verbindungen nach Anspruch 4, wobei R für Wasserstoff steht, n für 1 steht, X' für COOH steht, Y für COO⁻ steht und A wie in Anspruch 3 definiert ist.

6. Verbindungen nach Anspruch 5, wobei R für Wasserstoff steht, n für 1 steht, X' für COOH steht, Y für COO⁻ steht und A für Octreotid oder Octreotat steht.

7. Verbindungen nach An spruch 4, wobei R für COOH steht, n für 1 steht, X' für COOH steht, Y für COO⁻ steht und A wie in Anspruch 3 definiert ist.

8. Verbindungen nach Anspruch 7, wobei R für COOH steht, n für 1 steht, X' für COOH steht, Y für COO⁻ steht und A für Octreotid oder Octreotat steht.

9. Verbindungen nach Anspruch 4, ausgewählt aus der aus DOTAtyr ³-Octreotid, DOTAtyr ³-Octreotat, DOTA3tyr³-Octreotid, DOTA3tyr ³-Octreotat, DOTAt3tyr³-Octreotid und DOTAta.13tyr ³-Octreotat bestehenden Gruppe.

10. Verwendung von Verbindun gen nach Ansprüchen 1-3 zur Herstellung von Verbindungen nach Ansprüchen 4-9.

11. Verfahren zur Herstellung von mit radioaktiven Metallen markierten biologisch aktiven Molekülen, bei dem man:
a) Verbindungen nach Ansprüchen 1-3 mit geschützten Seitenketten an den N-Atomen und einer freien Carboxylatgruppe synthetisiert;
b) ein biologisch aktives Molekül an die freie Carboxylatgruppe kuppelt;
c) die geschützten Seitenketten entschützt und
d) die so erhaltene chelatbildende Struktur mit einem gewünschten radioaktiven Metall markiert.

12. Verbindungen nach Ansprüchen 4-9, markiert mit einem radioaktiven Metall zur Verwendung in der Diagnose und Therapie.

13. Verwendung von mit einem radioaktiven Metall markierten Verbindungen nach Ansprüchen 4-9 zur Herstellung einer diagnostischen oder therapeutischen Zusammensetzung zur Behandlung verschiedener Erkrankungen.

14. Verwendung nach Anspruch 13, wobei es sich bei der radioaktiven Metallmarkierung um ⁹⁰Y handelt.

## Revendications

1. Composés polyazamacrocycliques pour marquage par un métal radioactif, comprenant un système Nₙ, dans lequel n vaut 4, 5 ou 6, avec une taille de cycle variable, et dans lequel au moins un des atomes N est substitué par un groupe carboxylate libre destiné à un couplage à une fonction amino dans une molécule effectrice bioactive, tandis que tous les atomes N portent une chaîne latérale protégée.

2. Composé selon la revendication 1 répondant à la formule générale :

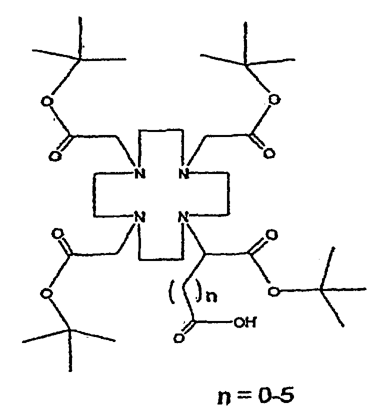

3. Composé selon la reven dication 1 ou 2, ce composé étant le 1-(1-carboxy-3-carbo-t-butoxypropyl)-4,7,10-(carbo-t-butoxyméthyl)-1,4,7,10-tétraazacyclododécane (DOTAGA(tBu)4).

4. Composés chélatants pour marquage de molécules bioactives avec un métal radioactif, répondant à la formule générale : 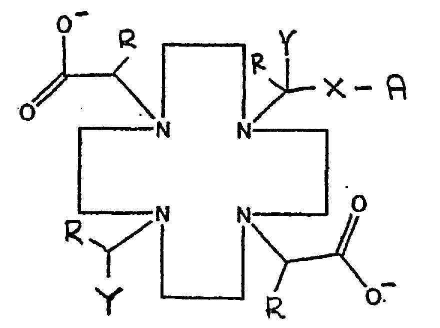 dans laquelle :
les deux groupes Y peuvent être placés en trans, comme indiqué, ou en cis ;
A est une molécule effectrice, telle qu'un peptide, en particulier un octréotide, le CCK, la substance P, la
gastrine, une protéine, en particulier un anticorps ou une enzyme, des sucres ou des agents radio-sensibilisants, comme la doxorubicine ;
R est un atome d' hydrogène, un groupe alkyle en C₁₋₃ ou un alcool ;
X est un segment intercalaire, en particulier (CH₂)ₙ-X', dans lequel n vaut 1-10 et X' est COOH, NH₂, SH, OH ou O-halogéno, où le groupe halogéno est en particulier Br, I ou Cl,
ou une molécule de formule 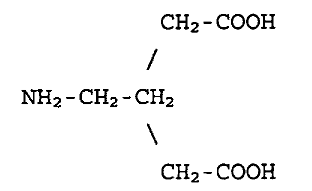 ou de formule 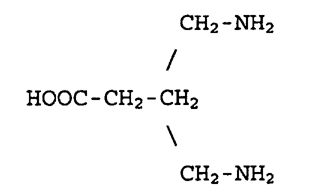 Y est COO⁻, CH₂CONH₂, CH₂CH₂OH,
éventuellement complexée avec un métal radioactif.

5. Composés selon la revendication 4, dans lesquels R est un atome d'hydrogène, n vaut 1, X' est COOH, Y est COO⁻ et A est tel que défini dans la revendication 3.

6. Composé selon la revendication 5, dans lequel R est un atome d'hydrogène, n vaut 1, X' est COOH, Y est COO⁻ et A est un octréotide ou un octréotate.

7. Composé selon la revendication 4, dans lequel R est COOH, n vaut 1, X' est COOH, Y est COO⁻ et A est tel que défini dans la revendication 3.

8. Composé selon la revendication 7, dans lequel R est COOH, n vaut 1, X' est COOH, Y est COO⁻ et A est un octréotide ou un octréotate.

9. Composés selon la revendication 4, choisis dans le groupe constitué par le DOTAtyr ³octréotide, le DOTAtyr³octréotate, le DOTA3tyr³octréotide, le DOTA3tyr³⁻octréotate, le DOTAt3tyr ³octréotide, le DOTAta.13tyr ³⁻octréotate.

10. Utilisation de composés selon les revendications 1-3 pour la préparation de composés selon les revendications 4-9.

11. Procédé de préparatio n de molécules bioactives marquées par un métal radioactif, comprenant les étapes consistant à :
a) synthétiser des composés selon l'une quelconque des revendications 1-3 portant des chaînes latérales protégées sur les atomes N et un groupe carboxylate libre ;
b) coupler une molécule bioactive au groupe carboxylate libre ;
c) déprotéger les chaînes latérales protégées ; et
d) marquer la structure du chélateur ainsi obtenu avec un métal radioactif souhaité.

12. Composés selon les revendications 4-9 marqués avec un métal radioactif à utiliser en diagnostic et en thérapeutique.

13. Utilisation de composés selon les revendications 4-9 marqués avec un métal radioactif pour la préparation d'une composition diagnostique ou thérapeutique destinée au traitement de différentes maladies.

14. Utilisation selon la revendication 13, dans laquelle le marqueur métallique radioactif est ⁹⁰Y.
